# DEMANDE DE BREVET EUROPEEN

(11) **EP 0 764 659 A1**
(43) Date de publication de la demande: **26.03.1997**
(21) Numéro de dépôt: 96401982.2
(22) Date de dépôt: 18.09.1996
(51) Int. Cl.: C08B 37/16

(54) **Utilisation de cyclodextrines et de leurs dérivés pour la solubilisation de composés de la famille des Ginkgolides**

(30) Priorité: 20.09.1995 FR 9511040
(71) Demandeur: COMMISSARIAT A L'ENERGIE ATOMIQUE, 75015 Paris (FR)
(72) Inventeur: Defaye, Jacques, 38330 Saint Ismier (FR); Laine, Valérie, 38440 St Jean de Bournay (FR); Djedaini-Pilard, Florence, 91150 Etampes (FR); Perly, Bruno, 78320 La Verriere (FR)
(74) Mandataire: Des Termes, Monique

(57) **Abrégé**

L'invention concerne l'utilisation de cyclodextrines naturelles et de leurs dérivés pour la solubilisation d'agents anti-agrégants plaquettaires de la familles des Ginkgolides.

Les cyclodextrines utilisées répondent à la formule : 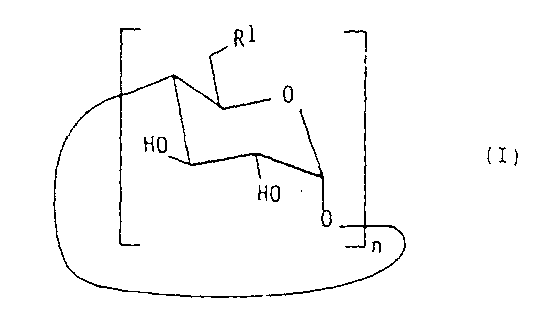 dans laquelle n est égal à 6, 7 ou 8, et les R¹ qui peuvent être identiques ou différents représentent OH ou SR² avec R² représentant un groupe dérivé d'un monsaccharide ou d'un oligosaccharide.

Le Ginkgolide est de préférence le Ginkgolide B.

## Description

La présente invention a pour objet un procédé de solubilisation dans un milieu aqueux d'un agent antagoniste du facteur d'agrégation des plaquettes appartenant à la famille des Ginkgolides.

Les Ginkgolides sont des composés chimiques comportant 6 cycles à 5 chaînons accolées.

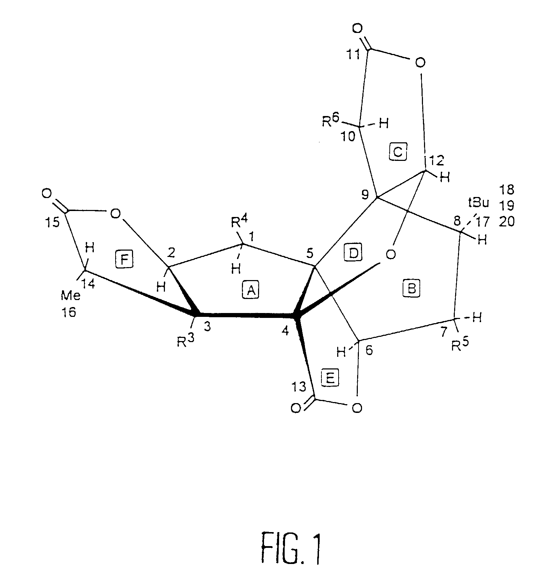

Sur la figure 1 annexée, on a représenté la formule développée de ces Ginkgolides. Ainsi, on voit que les Ginkgolides comportent un cycle tétrahydrofurane (D), 2 cycles cyclopentane ayant un carbone commun (A et B) formant un spiro-(4,4) nonane et 3 γ-lactones (C, E et F). Les Ginkgolides sont extraits des feuilles du *Ginkgo biloba,* ils ont une structure unique en forme de cage et présentent un fort potentiel d'activités biologiques. La grande diversité de ces activités dans la série des Ginkgolides semble être uniquement due aux différences de substitution et à des changements conformationnels.

Dans ces Ginkgolides dont la formule est donnée sur la figure 1, R³, R⁴, R⁵ et R⁶ peuvent représenter H ou OH ; R⁴ peut de plus représenter un groupe alcoxy inférieur tel que méthoxy (OMe) ou éthoxy (OEt).

Le tableau 1 qui suit regroupe certains Ginkgolides connus répondant à cette formule avec la nature des substituants R³, R⁴, R⁵ et R⁶.

**TABLEAU 1**

| Composé N° | Ginkgolide | Nomenclature IHB | R³ | R⁴ | R⁵ | R⁶ |
|---|---|---|---|---|---|---|
| 1 | A | BN 52020 | OH | H | H | OH |
| 2 | B | BN 52021 | OH | OH | H | OH |
| 3 | C | BN 52022 | OH | OH | OH | OH |
| 4 | J | BN 52024 | OH | H | OH | OH |
| 5 | M | BN 52023 | H | OH | OH | OH |
| 6 | Synthétique | BN 50580 | OH | OMe | H | OH |
| 7 | Synthétique | BN 50585 | OH | OEt | H | OH |

Les Ginkgolides majoritaires sont les composés 1, 2 et 3, formes A, B et C, les autres formes (J,M), composés 4 et 5, étant rares. Le composé ayant les propriétés pharmacologiques les plus intéressantes est le Ginkgolide B (composé 2) avec les 3 cycles lactoniques fermés.

Le Ginkgolide B a été le sujet d'un nombre considérable de travaux dans des domaines variés (Braquet, P., "Ginkgolides : Chemistry, Biology, Pharmacology and Clinical Perspectives", 1 et 2 ; J.R. Prous Science Publishers : Barcelone 1988). De plus, la rétrosynthèse complète a été effectuée par Corey (Corey, E.J., Kang M.C., Ghosh, A.K. ; Houpis, I.N. ; J. Am. Chem. Soc., 1988, 110, 649-651). Le Ginkgolide B présente des propriétés pharmacologiques intéressantes et semble montrer une activité antagoniste contre le facteur d'agrégation des plaquettes (anticoagulant) P.A.F. (Platelet Activating Factor) qui joue un rôle clé dans le processus inflammatoire. Il possède donc une activité anticoagulante. *In vitro*, des études pharmacologiques ont mis en évidence que l'efficacité du Ginkgolide B était étroitement liée au pH et que l'activité anti-PAF était augmentée en milieu acide (Braquet, P, Op. Cit. 1, 11-23). Ce résultat est clairement en relation avec l'existence de trois groupements lactone qui peuvent être hydrolysés, ce qui conduit à l'ouverture des cycles. Ces processus sont réversibles car, comme l'a montré Braquet, l'acidification des solutions basiques de Ginkgolide B régénère toujours le Ginkgolide B de départ sans aucune altération de la structure moléculaire. Ce point important a été confirmé par RMN (voir Thèse Oussama ZEKRI, Université de Rennes, 1994). Les auteurs ont montré la présence de 8 formes possibles dont l'existence dépend étroitement de la valeur du pH. Ainsi trois valeurs de pKa ont pu être déterminées à 7,14, 8,60 et 11,90. Dans des conditions physiologiques (solution tampon de phosphate physiologique (PBS), pH = 7,39), la solubilité du Ginkgolide B est de 0,2 mM et l'étude par RMN a montré la présence de 4 formes différentes, la forme fermée (forme active) représentant 34 % de l'ensemble à 25°C. On peut noter que ce pourcentage diminue lorsque la température augmente.

Le Ginkgolide B peut être administré par voie parentérale. En raison de sa faible solubilité à pH acide ou neutre, il est solubilisé à pH = 8,75 à une concentration de 20 mg/mL (C = 47mM) en présence de 10 mg/mL de mannitol. L'étude par RMN du proton à 500 MHz de cette solution à mis en évidence la présence de 5 formes ouvertes représentant plus de 99 % du Ginkgolide B. La forme totalement fermée, la seule active, est en proportion négligeable c'est-à-dire à une concentration inférieure à 0,1 mM. De plus, il a été montré par conductimétrie, que si l'ouverture des lactone était relativement rapide (≈ 10 min.), leur fermeture, elle, est lente (≥ 3 h) (voir la thése Oussama ZEKRI, Université de Rennes, 1994). On peut donc dire que sous la forme actuelle d'injection, le pourcentage de forme active anti PAF est extrêmement faible alors que la quantité de Ginkgolide injecté est importante. Pour résoudre ce problème une approche, qui fait justement l'objet de la présente invention, a été de trouver des molécules appropriées présentant une solubilité élevée dans un milieu aqueux, à pH physiologique, pour solubiliser les Ginkgolides tels que le Ginkgolide B, en milieu aqueux, tout en conservant le plus possible la forme active, c'est-à-dire la forme fermée du Ginkgolide.

La présente invention a précisément pour objet l'utilisation de cyclodextrines naturelles et de certains de leurs dérivés, pour solubiliser et protéger dans un milieu aqueux des agents anti-PAF du type Ginkgolides par inclusion de ceux-ci dans ces cyclodextrines.

Les cyclodextrines ou cyclomaltooligosaccharides sont des composés d'origine naturelle formés par l'enchaînement de 6, 7 ou 8 unités glucose liées en α-(1→4). De nombreux travaux ont montré que ces cyclodextrines pouvaient former des complexe d'inclusion avec des molécules hydrophobes et permettre ainsi la solubilisation de ces molécules dans des milieux aqueux. De nombreuses applications ont été proposées pour tirer profit de ce phénomène, en particulier dans le domaine pharmaceutique, comme il est décrit par D. Duchêne dans l'ouvrage intitulé "Cyclodextrins and their industrial uses", chapitre 6, pages 213 à 257, Editions de Santé, 1987. Des compositions pharmaceutiques utilisant des cyclodextrines ont d'ailleurs été commercialisées au Japon, en Italie et plus récemment en France, par exemple par Pierre Fabre Médicament pour le Brexin® qui est un complexe d'inclusion du Piroxicam dans la β-cyclodextrine.

Selon l'invention, le procédé de solubilisation dans un milieu aqueux d'un agent antagoniste du facteur d'agrégation des plaquettes appartenant à la famille des Ginkgolides consiste à combiner le dit agent avec une cyclodextrine de formule : 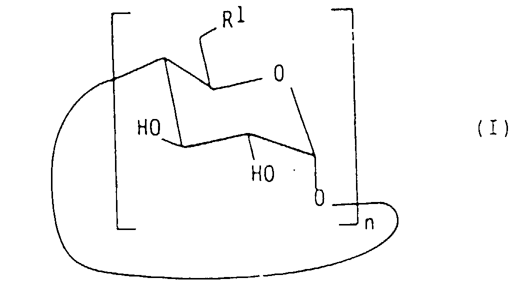 dans laquelle n est égal à 6, 7 ou 8, et les R¹ qui peuvent être identiques ou différents, représentent OH ou SR² avec R² représentant un groupe dérivé d'un monosaccharide ou d'un oligosaccharide, pour former avec celle-ci un complexe d'inclusion soluble dans l'eau.

Dans la formule (I) donnée ci-dessus, R² peut répondre à la formule : 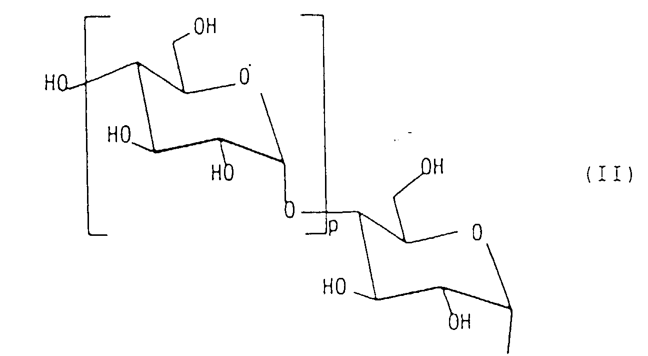 dans laquelle p est égal à 0 ou est un nombre entier de 1 à 5.

Selon l'invention, les agents antagonistes du facteur d'agrégation des plaquettes, dénommés ci-après agents anti-PAF, sont des composés appartenant à la famille des Ginkgolides. Ils peuvent répondre à la formule donnée sur la figure 1, dans laquelle R³, R⁵ et R⁶ qui peuvent être identiques ou différents, représentent H ou OH, et R⁴ représente H, OH ou un groupe alcoxy de 1 à 5 atomes de carbone.

Les groupes alcoxy utilisés peuvent être en particulier les groupes méthoxy et éthoxy.

De préférence l'invention s'applique à l'agent anti-PAF constitué par le Ginkgolide B répondant à la formule (III) dans laquelle R³, R⁴ et R⁶ représentent OH et R⁵ représente H.

Selon un premier mode de réalisation de l'invention, la cyclodextrine est une cyclodextrine naturelle répondant à la formule (I) avec tous les R¹ représentant OH. De préférence, dans ce mode de réalisation, n est égal à 7.

Selon un second mode de réalisation de l'invention, on utilise une cyclodextrine ramifiée, c'est-à-dire une cyclodextrine répondant à la formule (I) donnée ci-dessus, dans laquelle un seul R¹ représente SR², les autres R¹ étant identiques et réprésentant OH.

Dans ce second mode de réalisation, R² est de préférence le groupe α-maltosyle ou β-maltosyle, soit le groupe répondant à la formule (II) avec p égal à 1.

Comme dans le premier mode de réalisation, on préfère les cyclodextrines ramifiées de formule (I) dans laquelle n est égal à 7. ou 8, les meilleurs résultats étant obtenus avec n égal 7 ou 8, plutôt 7.

Les dérivés de cyclodextrine utilisés dans ce second mode de réalisation de l'invention peuvent être préparés comme il est décrit dans EP-A-0 403 366 et FR-A-2 715 307.

L'invention a également pour objet, les complexes d'inclusion d'un agent antagoniste du facteur d'agrégation des plaquettes, appartenant à la famille de Ginkgolides, dans une cyclodextrine répondant à la formule (I) précitée.

Dans ces complexes d'inclusion, on préfère également que la cyclodextrine de formule (I) soit la β-cyclodextrine (n égal 7) ou la γ-cyclodextrine (n égal 8) avec tous les R¹ identiques représentant OH, ou un dérivé de la β-cyclodextrine dans lequel un seul R¹ représente SR² avec R² représentant le groupe α-maltosyle ou β-maltosyle, les autres R¹ étant identiques et représentant OH.

Les agents antagonistes inclus dans la cyclodextrine de ce complexe peuvent être les Ginkgolides de formule (III) décrits sur la figure 1 et dans le tableau 1 ci-dessus, en particulier le Ginkgolide B qui correspond à la formule (III) avec R³, R⁴ et R⁶ représentant OH et R⁵ représentant H.

Ces complexes d'inclusion peuvent être préparés de façon classique, par exemple en ajoutant à une solution de la cyclodextrine de formule (I) précitée, dont le pH est inférieur à 5, une solution concentrée de Ginkgolide dans un solvant organique approprié, par exemple l'acétone. Il est à noter que la valeur du pH de la solution aqueuse est primordiale. En effet à un pH inférieur à 5, les Ginkgolides, par exemple le Ginkgolide B, sont uniquement sous la forme entièrement fermée, qui est la seule forme active dans le cas du Ginkgolide B. On peut ensuite isoler le complexe d'inclusion ainsi formé par lyophilisation.

Pour éviter l'utilisation d'un solvant organique, lors de la préparation du complexe d'inclusion, on peut disperser l'agent anti-PAF à inclure dans une solution aqueuse de la cyclodextrine utilisée dont le pH est inférieur à 5, pour les mêmes raisons que décrit précédemment, et agiter la suspension obtenue jusqu'à l'obtention d'une solution claire. On peut ensuite isoler le complexe d'inclusion ainsi formé par lyophilisation comme précédemment.

Ces complexes d'inclusion peuvent être utilisés en particulier dans des compositions pharmaceutiques qui comprennent de plus un véhicule pharmaceutique acceptable.

Ces compositions pharmaceutiques qui peuvent être administrées par voie orale ou parentérale, sont par exemple des solutions, des poudres, des suspensions, en particulier des solutions injectables. Les complexes d'inclusion formés avec le Ginkgolide B et les cyclodextrines naturelles ou ramifiées ont une solubilité à pH acide, supérieure à celle du Gingolide B seul. De plus à des pH plus élevés, le Ginkgolide B sous forme de complexe d'inclusion, voit sa proportion de forme fermée augmenter de façon significative par rapport au Ginkgolide B seul dans les mêmes conditions. Ainsi, l'utilisation de cycldextrines naturelles ou ramifiées permet de diminuer la quantité totale de Ginkgolide B injectée tout en augmentant la quantité de forme active, c'est-à-dire la proportion de forme fermée. En d'autres termes, le complexe d'inclusion est plus efficace vis-à-vis du P.A.F. tout en minimisant les risques d'effets secondaires.

D'autres caractéristiques et avantages de l'invention apparaîtront mieux à la lecture des exemples suivants, donnés bien entendu à titre illustratif et non limitatif, en référence aux dessins annexés.
- La figure 1 déjà décrite représente la formule des Ginkgolides utilisés dans l'invention.
- La figure 2 est une représentation des spectres partiels de résonance magnétique nucléaire (RMN) du proton des complexes d'inclusion préparés dans les exemples 1, 2 et 3 et du Ginkgolide B seul.

### EXEMPLE 1 : Préparation d'un complexe d'inclusion du Ginkgolide B avec la β-cyclodextrine.

On part d'une solution concentrée de Ginkgolide B dans un solvant miscible à l'eau, constitué par de l'acétone, et on ajoute la quantité de solution concentrée qui correspond à 1 µmol de Ginkgolide B, à 1 mL d'une solution à 10 mmol/L de β-cyclodextrine dans l'eau stérile à pH = 4,9 et à la température ambiante. On élimine l'acétone sous barbotage d'azote et on lyophilise la solution. Le solide résiduel qui contient 10 µmol de dérivé de β-cyclodextrine et 1 µmol de Ginkgolide B est redissous dans un minimum d'eau à 25°C. Ce minimum correspond à 1 mL d'eau, ce qui indique une solubilité maximale de 1 mmol/L en Ginkgolide B dans l'eau en présence de β-cyclodextrine à une concentration de 10 mmol/L.

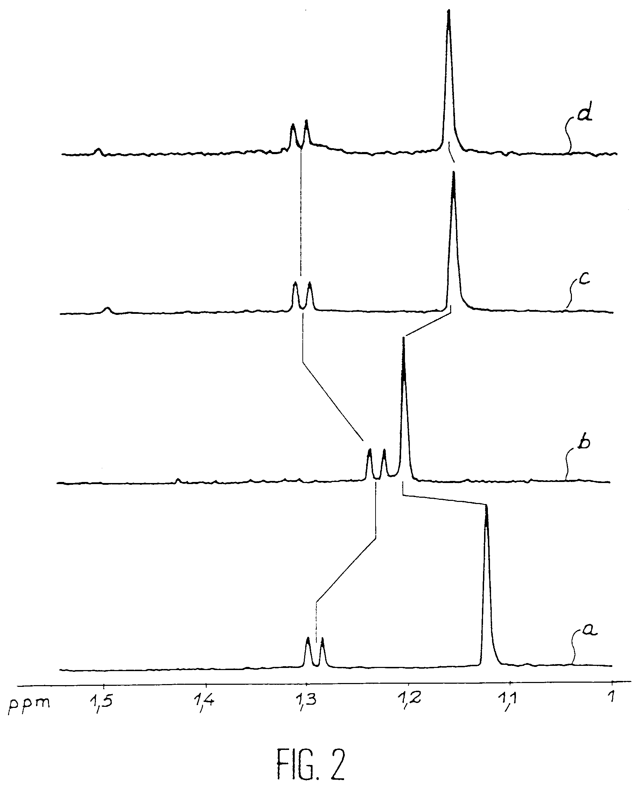

Le spectre partiel de Résonance Magnétique Nucléaire (RMN) du proton de ce complexe, à une concentration de 5 mmol/L dans D₂O, à 298K et à 500 MHz, est représenté sur la figure 2 (Spectre c). Sur cette figure, on a aussi représenté, à titre indicatif, le spectre partiel du Ginkgolide B seul (Spectre a) . En comparant les deux spectres, on remarque des modifications qui confirment l'inclusion du Ginkgolide B dans la cavité de la β-cyclodextrine.

De plus, on a observé que le Ginkgolide B n'existe dans le complexe d'inclusion que sous la forme fermée, à pH = 4,9.

Il est à noter que la solubilité du complexe d'inclusion est la même à pH physiologique, soit une solubilité maximale de 1 mmol/L de Ginkgolide B en présence de 10 mmol/L de β-cyclodextrine. Le Ginkgolide B seul, à pH physiologique, existe sous 4 formes différentes dont la forme fermée ne représente que 34 % à 25°C. Avec le complexe d'inclusion préparé comme décrit précédemment, on n'observe que deux formes différentes en proportions identiques dont la forme fermée. Par comparaison la solubilité maximale du Ginkgolide B seul, dans sa forme fermée, est de 0,2 mmol/l à pH = 4,9 et de 0,68 mmol/L à pH = 7,39. Lorsque le Ginkgolide B est sous forme de complexe d'inclusion, à pH 4,9, avec une concentration de 1 mmol/L en présence de 10 mmol/L de β-cyclodextrine, il a une concentration en forme fermée 10 fois supérieure à celle contenue dans la formulation injectable (1 mmol/L et 0,1 mmol/L respectivement) alors que la concentration totale en Ginkgolide B est 40 fois plus faible (1 mmol/L et 47 mmol/L respectivement).

### EXEMPLE 2 : Préparation d'un complexe d'inclusion du Ginkgolide B avec la γ-cyclodextrine.

On part d'une solution concentrée de Ginkgolide B dans un solvant miscible à l'eau, constitué par de l'acétone, et on ajoute la quantité de solution concentrée qui correspond à 1 µmol de Ginkgolide B, à 2 mL d'une solution à 5 mmol/L de γ-cyclodextrine dans l'eau stérile, à pH = 4,9 et à la température ambiante. On élimine l'acétone sous barbotage d'azote et on lyophilise la solution. Le solide résiduel qui contient 10 µmol de dérivé de γ-cyclodextrine et 1 µmol de Ginkgolide B est redissous dans un minimun d'eau à 25°C. Ce minimun correspond à 2 mL d'eau, ce qui indique une solubilité maximale de 0,5 mmol/L en Ginkgolide B dans l'eau en présence de γ-cyclodextrine à une concentration de 5 mmol/L.

Le spectre partiel de Résonance Magnétique Nucléaire (RMN) du proton de ce complexe à une concentration de 5 mmol/L dans D₂O, à 298K et à 5OO MHz est représenté sur la figure 2 (Spectre b) . En comparant ce spectre b au spectre a du Ginkgolide B seul, on remarque des modifications du spectre qui confirment l'inclusion du Ginkgolide B dans la cavité de la γ-cyclodextrine. De plus, on a observé que le Ginkgolide B n'existe dans le complexe d'inclusion que sous la forme fermée, à pH = 4,9.

Il est à noter que la solubilité du complexe d'inclusion est la même à pH physiologique soit 0,5 mmol/L du Ginkgolide en présence de 5 mmol/L de γ-cyclodextrine. Avec le complexe d'inclusion préparé comme décrit précédemment, on observe 4 formes différentes, la forme fermée représentant 70 %. Lorsque le Ginkgolide B est sous forme de complexe d'inclusion à pH 4,9 avec une concentrattion de 0,5 mmol/L en présence de 5 mmol/L de γ-cyclodextrine, il y a une concentration en forme fermée 5 fois supérieure à celle contenue dans la formulation injectable (0,5 mmol/L et 0,1 mmol/L respectivement) alors que la concentration totale en Ginkgolide B est 80 fois plus faible (0,5 mol/L et 40 mmol/L respectivement).

### EXEMPLE 3 : Préparation d'un complexe d'inclusion du Ginkgolide B avec le 6-S-α-maltosyl-6-thiocyclomaltoheptaose.

On part d'une solution concentrée de Ginkgolide B dans un solvant miscible à l'eau, constitué par de l'acétone, et on ajoute la quantité de solution concentrée qui correspond à 1 µmol de Ginkgolide B, à 1 mL d'une solution à 10 mmol/L du dérivé de β-cyclodextrine, le 6-*S*-α-maltosyl-6-thiocyclomaltoheptaose, dans l'eau stérile, à pH = 4,9 et à la température ambiante. On élimine l'acétone sous barbotage d'azote et on lyophilise la solution. Le solide résiduel qui contient 10 µmol de dérivé de β-cyclodextrine et 1 µmol de Ginkgolide B est redissous dans un minimum d'eau à 25°C. Ce minimum correspond à 500 µl d'eau, ce qui indique une solubilité maximale de 2 mmol/L en Ginkgolide B dans l'eau en présence de ce dérivé de β-cyclodextrine à une concentration de 20 mmol/L.

Le spectre partiel de Résonance Magnétique Nucléaire (RMN) du proton de ce complexe, à une concentration de 5 mmol/L dans D₂0, à 298K et à 500 MHz, est représenté sur la figure 2 (Spectre d). En comparant le spectre d au spectre a, on remarque des modifications du spectre qui confirment l'inclusion du Ginkgolide B dans la cavité du dérivé de la β-cyclodextrine. De plus, on a observé que le Ginkgolide B n'existe dans le complexe d'inclusion que sous la forme fermée à pH = 4,9.

Il est à noter que la solubilité du complexe d'inclusion est la même à pH physiologique soit 2 mmol/L de Gingolide B en présence de 20 mmol/L de dérivé de la β-cyclodextrine. Avec le complexe d'inclusion préparé comme décrit précédemment, on n'observe que deux formes différentes en proportions identiques dont la forme formée. Lorsque le Ginkgolide B est sous forme de complexe d'inclusion, à pH 4,9, avec une concentration de 2 mmol/L en présence de 20 mmol/L de 6-*S*-α-maltosyl-6-thiocyclomaltoheptaose, il a une concentration en forme fermée 20 fois supérieure à celle contenue dans la formulation injectable (2 mmol/L et 0,1 mol/ respectivement) alors que la concentration totale en Ginkgolide B est plus faible (2 mmol/L et 40 mmol/L respectivement).

## Revendications

1. Procédé de solubilisation dans un milieu aqueux d'un agent antagoniste du facteur d'agrégation des plaquettes, appartenant à la famille des Ginkgolides, caractérisé en ce qu'il consiste à combiner ledit agent avec une cyclodextrine de formule : 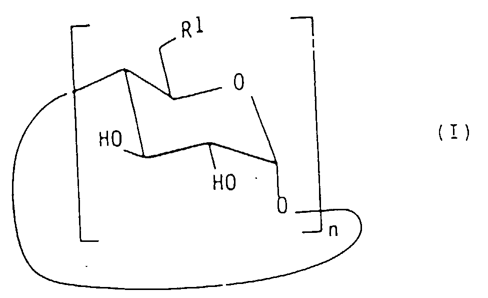 dans laquelle n est égal à 6, 7 ou 8, et les R¹ qui peuvent être identiques ou différents représentent OH ou SR² avec R² représentant un groupe dérivé d'un monosaccharide ou d'un oligosaccharide, pour former avec celle-ci un complexe d'inclusion soluble dans l'eau.

2. Procédé selon la revendication 1, caractérisé en ce que R² répond à la formule : 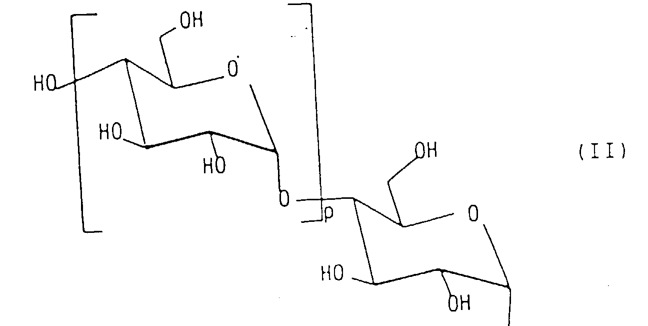 dans laquelle p est égal à 0 ou est un nombre entier de 1 à 5.

3. Procédé selon l'une quelconque des revendications 1 et 2, caractérisé en ce que l'agent antagoniste comprend un Ginkgolide de formule : 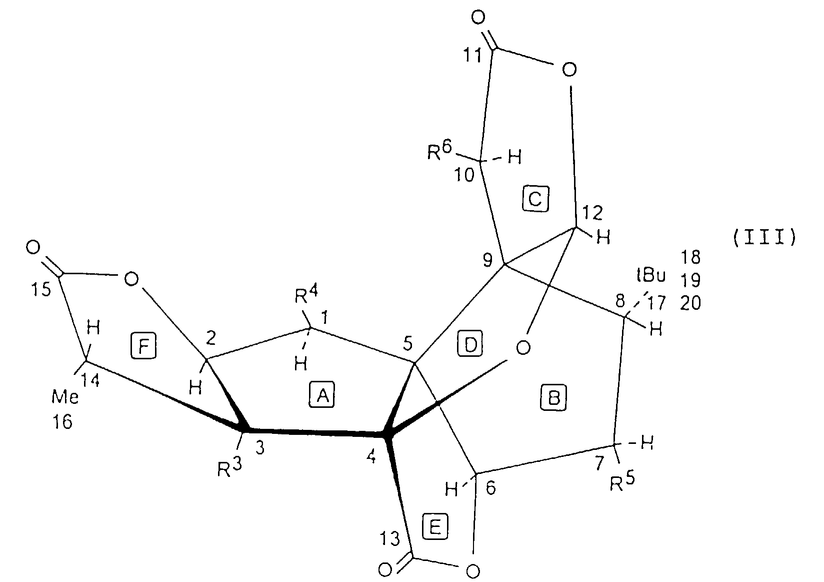 dans laquelle R³, R⁵ et R⁶ qui peuvent être identiques ou différents, représentent H ou OH, et R⁴ représente H, OH ou un groupe alcoxy de 1 à 5 atomes de carbone.

4. Procédé selon la revendication 3, caractérisé en ce que R³, R⁴ et R⁶ représentent OH et R⁵ représente H.

5. Procédé selon l'une quelconque des revendications 1 à 4, caractérisé en ce que tous les R¹ représentent OH.

6. Procédé selon l'une quelconque des revendications 1 à 4, caractérisé en ce que l'un des R¹ représente SR² avec R² étant un groupe α-maltosyle ou β-maltosyle, les autres R¹ représentant OH.

7. Procédé selon l'une quelconque des revendications 1 à 6, caractérisé en ce que n est égal à 7 ou 8.

8. Complexe d'inclusion d'un agent antagoniste du facteur d'agrégation des plaquettes, appartenant à la famille des Ginkgolides dans une cyclodextrine de formule : 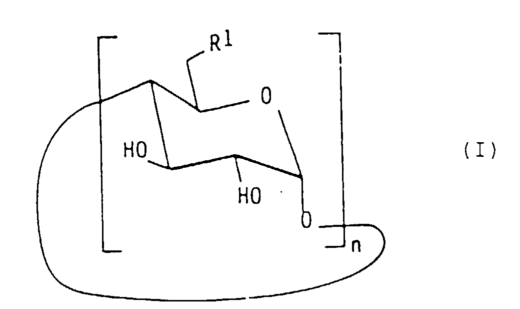 dans laquelle n est égal à 6, 7 ou 8, et les R¹ qui peuvent être identiques ou différents, représentent OH ou SR² avec R² représentant un groupe dérivé d'un monosaccharide ou d'un oligosaccharide,

9. Complexe selon la revendication 8, caractérisé en que R² répond à la formule (II) : 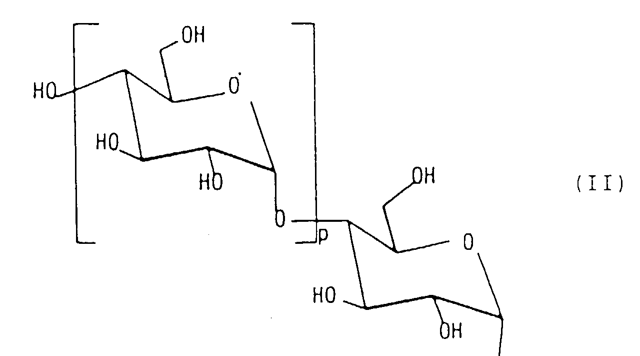 dans laquelle p est égal à 0 ou un nombre entier de 1 à 5.

10. Complexe selon l'une des revendication 8 et 9, caractérisé en que l'agent antagoniste répond à la formule : 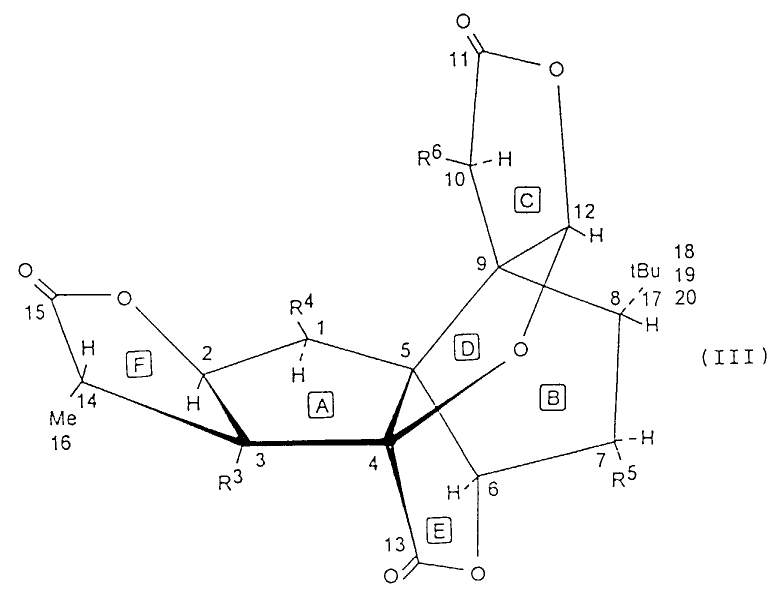 dans laquelle R³, R⁵ et R⁶ qui peuvent être identiques ou différents, représentent H ou OH, et R⁴ représente H, OH, et R⁴ représente H, OH ou un groupe alcoxy de 1 à 5 atomes de carbone.

11. Complexe selon la revendication 10, caractérisé en que R³, R⁴ et R⁶ représentent OH et R⁵ représente H.

12. Complexe selon l'une quelconque des revendications 8, 10 et 11, caractérisé en que tous les R1 représentent OH et n est égal à 7 ou 8.

13. Complexe selon l'une quelconque des revendications 8, 10 et 11, caractérisé en que que n est égal à 7, et l'un des R¹ représente SR² avec R² étant le groupe α-maltosyle.

14. Composition pharmaceutique, caractérisée en ce qu'elle comprend un complexe d'inclusion selon l'une quelconque des revendications 8 à 13 avec un véhicule pharmaceutiquement acceptable.
